# EUROPEAN PATENT APPLICATION

(11) **EP 4 674 340 A1**
(43) Date of publication of application: **07.01.2026**
(21) Application number: 24185652.5
(22) Date of filing: 01.07.2024
(51) Int. Cl.: A61B 3/14, H03M 1/18

(54) **OPHTHALMIC IMAGING INSTRUMENT AND LIGHT BEAM POWER CONTROL THEREIN**

(71) Applicant: Optos plc, Dunfermline, Scotland KY11 8GR (GB)
(72) Inventor: GEDDES, David, Dunfermline, Scotland KY11 8GR (GB)
(74) Representative: Hoffmann Eitle

(57) **Abstract**

An ophthalmic imaging instrument comprising a light source; a controller arranged to control the light source to generate a beam of light of a target optical power; a beam splitter arranged to split the beam of light into a first beam of light, and a second beam of light which propagates towards an eye; a detector arranged to receive the first beam of light and generate a signal indicative of an optical power of the first beam of light; and a signal converter arranged to generate an output based on the signal indicative of the optical power, the signal converter having a variable gain for generating the output, wherein the controller is further arranged to control the gain of the signal converter using the target optical power such that the signal converter generates a predefined output when an optical power of the beam of light is the target optical power.

## Description

### Technical field

The present invention relates to an ophthalmic imaging instrument, and more particularly but not exclusively, to controlling the optical power of one or more light sources that contribute to a beam of light used for imaging an eye of a subject.

### Background

Ophthalmic imaging instruments are widely used to image a patient's eye in order to assess the health of the eye. Such systems comprise a light source and a controller that is arranged to control the light source to generate a beam of light of a target optical power. A beam splitter is usually provided for splitting the beam of light into two, wherein one partial beam propagates towards an eye of a subject and the other partial beam is directed at a detector which translates the measured power into some signal that is indicative of the optical power incident into the detector. Assuming a well-defined and constant splitting ratio of the beam splitter, the optical power that is actually directed toward an eye can be controlled by means of controlling the output power of the light source on the basis of the signal being indicative of the optical power of the partial beam that is incident into the detector.

As in ophthalmic imaging the imaging target is a subject's eye, appropriate power control is essential. On the one hand, the optical power of light incident into the eye must not exceed a given power budget at any time, as otherwise the light may damage tissue. On the other hand, however, the optical power needs to be high enough in order to obtain a sufficient imaging quality. Notably, the signal to noise ratio may become undesirably low if the optical power of the incident beam is too weak. Therefore, common ophthalmic imaging instruments comprise control mechanisms that keep the optical power inside a permissible power band, i.e. below a maximum threshold power in order to prevent damage to a subject's eye, and above a minimum threshold in order to provide the desired imaging quality.

Such control mechanisms usually shut the light source down or prevent the light from reaching a subject's eye by other means whenever the light beam power moves outside the permissible band. Given the significance of keeping the light beam power inside a permissible power band, most respective control mechanisms are implemented as fail-proof as possible, resulting in the desired high reliability as an advantage, but, as a disadvantage, also in a poor flexibility when being operated under varying conditions or in varying imaging modalities.

Specifically, some modern ophthalmic imaging instruments can operate in multiple imaging modalities such that the clinician can select an imaging modality that is most suited to imaging a given region or disease in the patient's eye. Each imaging modality allows the ophthalmic imaging system to acquire a different type of ophthalmic image, which helps the clinician to acquire images containing information most appropriate for diagnosing ocular disease. For example, in a scanning laser ophthalmoscope (SLO), a colour imaging modality may be used to acquire a fundus image of a patient's ocular fundus, including a portion of the retina. In this imaging modality, a combination of red, green and blue laser light may be delivered to the patient's eye to acquire images of the ocular fundus. Infrared retinal imaging is another example of an imaging modality that may be used to image a patient's eye. Infrared imaging delivers light from an infrared laser to the patient's eye, and may be used to detect retinal pathologies such as intraretinal fluid or retinal pigment epithelium tear. Another example of an imaging modality is optical coherence tomography (OCT). OCT imaging is a non-invasive imaging technique that allows a clinician to acquire cross-sectional and/or three-dimensional images of tissue.

As power control remains essential even under varying imaging conditions or modalities, the existing power control mechanisms oftentimes fail to accommodate for both sufficient and reliable control of light beam powers as well as the desired flexibility. There is therefore a need for improved power control mechanisms in ophthalmic imaging instruments that allow for the flexibility but maintain safety while keeping system complexity at an acceptable level.

### Summary

Problems are solved and objects are met by the subject matter of the main independent claim 1. Further preferred embodiments are defined in the dependent claims.

According to one embodiment of the present invention, there is provided an ophthalmic imaging instrument comprising a light source; a controller arranged to control the light source to generate a beam of light of a target optical power; a beam splitter arranged to split the beam of light into a first beam of light, and a second beam of light which propagates towards an eye of a subject; a detector arranged to receive the first beam of light and generate a signal indicative of an optical power of the first beam of light; and a signal converter arranged to generate an output based on the signal indicative of the optical power, the signal converter having a variable gain for generating the output, wherein the controller is further arranged to control the gain of the signal converter using the target optical power such that the signal converter generates a predefined output when an optical power of the beam of light is the target optical power.

### Brief Description of the Drawings

Embodiments of the present invention, which are presented for better understanding of the inventive concepts, but which are not to be seen as limiting the invention, will now be described with reference to the figures in which:
- Figure 1: shows a schematic view of an ophthalmic imaging instrument according to a general device embodiment of the present invention;
- Figure 2: shows a schematic view of a protection circuit as part of an ophthalmic imaging instrument according to a more specific device embodiment of the present invention;
- Figures 3A & 3B: show schematic views, including circuit diagrams, of signal converters as part of an ophthalmic imaging instrument according to more specific device embodiments of the present invention;
- Figures 4A & 4B: show schematic views of ophthalmic imaging instruments for providing more than one imaging modality according to more specific device embodiments of the present invention.
- Figure 5: shows schematic views of optical powers for individual beams of light under different imaging modalities according to a more specific embodiment of the present invention; and
- Figure 6: shows a flowchart of a general method embodiment of the present invention.

### Detailed Description

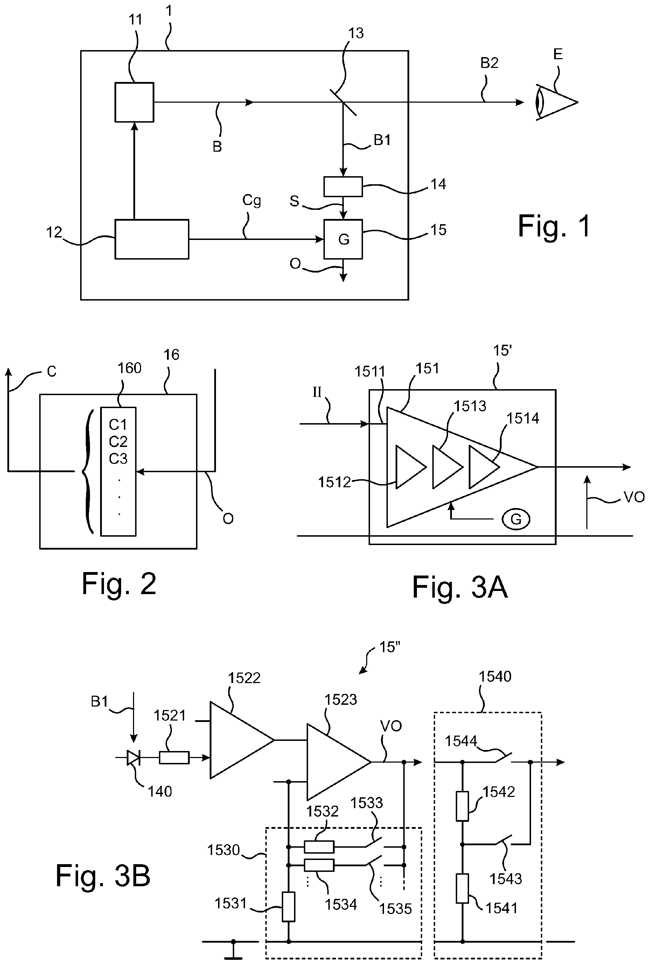

Figure 1 shows a schematic view of an ophthalmic imaging instrument according to a general device embodiment of the present invention. The shown ophthalmic imaging instrument is intended for imaging a subject's (patient's) eye in order to - for example - assess the health of the eye. The present embodiment relates in particular, but not exclusively, to controlling the optical power of one or more light sources that contribute to a beam of light used for imaging an eye of a subject. The shown ophthalmic imaging instrument 1 comprises a light source 11 and a controller 12 which is arranged to control the light source 11 to generate a beam of light B of a target optical power. For example, the light source 11 can comprise or be a laser of a well-defined and suitable colour. The controller 12 may be arranged to regulate the optical power of the beam of light by minimizing an error signal which is indicative of a difference between a figure that represents the measured optical power and a figure that represents the target optical power of the beam of light. A beam splitter 13 is arranged to split the beam B of light into a first beam of light B1, and a second beam of light B2 which propagates towards an eye E of a subject or patient. The ophthalmic imaging instrument 1 further comprises a detector 14 which is arranged to receive the first beam of light B1 and generate a signal S which is indicative of an optical power of the first beam of light B1.

The signal S as a detector output is input to a signal converter 15 which is arranged to generate an output O based on the signal S indicative of the optical power of the first beam of light B1 which is incident from the beam splitter 13 into the detector 14. The beam splitter 13 is preferably arranged to split the beam of light B into the two beams of light B1, B2 such that their respective power ratio remains well defined and constant over respectively applicable ranges of optical power, light wavelengths, operation temperatures, operation times, and/or other applicable figures that are relevant when operating the ophthalmic imaging instrument 1 in an appropriate and compliant manner. Specifically, the ophthalmic imaging instrument 1 and - in particular-the beam splitter 13 may consider that the optical power of the first beam of light B1 is detected in order to control the optical power of the second beam of light B2 which propagates toward the eye E of a subject. The significance of this aspect mainly originates from the fact that the optical power that is actually incident into parts of the eye E is to conform to well defined power budgets in order to avoid any damage to the eye E but also ensure sufficient imaging quality as described also elsewhere in the present disclosure.

The signal converter 15 has a variable gain G for generating the output O, the gain G being generally a figure that governs a conversion characteristic of the signal converter 15 under operation and under conversion of the signal S and an input to the output O as an output signal. The input signal S and/or the output signal O can be provided, routed and/or processed in any suitable manner, including but not limited to an analog form, a digital form, an optical form, an electromagnet wave and the like. For example, an analog signal may be provided, routed and/or processed in the form of an electric voltage or an electric current. In such cases the amplitude of the voltage or current usually corresponds to the amplitude of the observable, e.g. the optical power of a beam of light, in a well-defined and constant manner. As a further example, a digital signal may be provided, routed and/or processed in the form of a serial or parallel stream of bit streams, in which respective information bits are represented by specific electric voltages or currents or features in an optical or other electromagnetic wave signal. Suitable digital signal protocols include but are not limited to I2C, SPI, one-wire, and the like. In the cases of a digital signal handling, the amplitude of the observable, e.g. the optical power of a beam of light, is represented by a numerical value in a well-defined and constant manner, wherein said numerical value is in turn represented by a number of bits again in a well-defined manner. For example, the I2C concept allows for handling numerical values in chunks of one byte in the form of 7 or 8 bits.

The gain G of the signal converter 15 is variable so that it can be changed in order to change the conversion characteristic of the signal converter 15. For example, the gain G can govern the conversion of an input electric current (input signal S) to an output electric voltage (output signal O). For example, a first gain G1 can yield a conversion of an input current of 1mA to an output voltage of 1 V, whereas a second gain G2 can yield a conversion of the input current of 1mA to an output voltage of 0.3 V (volt). The gain G can be adjusted by means of again an analog or digital control signal. For example, the signal converter 15 may comprise a control input that is arranged to receive a control signal Cg that controls and determines the gain G of the signal converter 15. The amplitude of the control signal or, respectively, the numerical value represented by a digital control signal may correspond to the gain G in a well-defined and constant manner. In digital implementations this may also include the setting and control of further conversion characteristics such as linearity, averaging, sample rates and the like. Further, the gain G may also be varied in steps in the form of selecting one out of several gains. For example, such a selection may consider the above-mentioned gains G1 and G2 as being available for selection. Also, a set of e. g. equidistant gains may be provided so that an overall gain G may be achieved over selecting individual gains ins multiple steps.

In the ophthalmic imaging instrument 1 according to the shown general device embodiment the controller 12 is further arranged to control the gain G of the signal converter 15 using the target optical power such that the signal converter 15 generates a predefined output when an optical power of the beam of light is the target optical power. In this way, the signal converter 15 can be tuned to output some target output for different and varying optical powers. Specifically, the predefined output may be independent from the target optical power. For example, the controller 12 can control the gain of the signal converter 15 to a first gain G1 in which the signal converter 15 outputs a predefined output, for example 1V in the case of an analog voltage output, when the input signal S is indicative of an optical power of 2 mW, whereas the controller 12 can control the gain of the signal converter 15 to a second gain G2 in which the signal converter 15 again outputs the predefined output, for example 1 V, when the input signal S is indicative of an optical power of 0.6 mW. It is noted that optical powers are usually in a range of 2 mW to 5 mW in the context of the present applications.

Such control may substantially improve the operation of the ophthalmic imaging instrument 1 under varying operation schemes, including but not limited to varying imaging modalities. For example, the ophthalmic imaging instrument 1 can be operated in one mode or imaging modality in which an optical power of 2 mW of the light source corresponds to the target optical power, and in at least one other mode or imaging modality in which an optical power of 0.6 mW of the light source corresponds to the target optical power. Generally, the output O can be provided to other elements of the ophthalmic imaging instrument 1 or external elements and/or components for facilitating one or more specific aspects of operation control of the ophthalmic imaging instrument 1.

Figure 2 shows a schematic view of a protection circuit as part of an ophthalmic imaging instrument according to a more specific device embodiment of the present invention. In such embodiments, the ophthalmic imaging instrument 1 further comprises a protection circuit 16 which is arranged to determine whether the output generated by the signal converter 15 fails to satisfy at least one predetermined criterion, and to control the ophthalmic imaging instrument 1 to prevent delivery of the second beam B2 of light towards the eye E of the subject when the output O fails to satisfy the at least one predetermined criterion. As shown in the form of functional elements in Figure 2, the protection circuit 16 is arranged to receive the output O from the signal converter 15 as described in more detail elsewhere in the present disclosure. The output O can be directly provided to the protection circuit 16 from the signal converter 15 by means of, for example, a direct signal connection coupling the output voltage as the output signal O to the protection circuit 16. However, there may also be provided intermediate and/or further signal processing or conversion so that there is no direct connection or coupling, but the protection circuit 16 still receives an input that is indicative of the output signal O provided by the signal converter 15.

The protection circuit 16 may further provide a control signal C for controlling the ophthalmic imaging instrument 1 or specific parts and elements thereof to prevent delivery of the second beam B2 of light towards the eye E. For example, the control signal C may be conveyed toward the controller 12 which is generally arranged to control the light source 11 to generate the beam of light B of a target optical power. For example, the controller 12 may be configured so as to set the target optical power to O when the control signal C indicates that delivery of the second beam B2 of light towards the subject's eye is to be prevented. This may involve an intervention into the regular power control system so as to reduce the power to zero and, therefore, shut off the primary beam of light B.

However, also other mechanisms may apply, such as interrupting the power supply to the light source 11 and/or controlling any other elements arranged in the optical path between the light source 11 and the subject's eye E, such as shutters, filters, deflectors, mirrors and the like. In order to control the prevention of delivery of the beam of light to the eye E, the protection circuit 16 may be arranged to consider one or more criteria C1, C2, ... that can be individually or cumulatively assessed from the output signal O which is indicative of the optical power of the first beam of light B1, being in turn - by means of the already described beam splitter 13 - indicative of the optical power of the second beam of light B2 propagating toward the subject's eye E. A criterion may consider any one of a lower threshold, an upper threshold, a power band, an averaging, an integration of power overtime, a power dosage or budget and the like. The protection circuit 16 may comprise an evaluator 160, for example in the form of a state machine or other suitable logic circuit, that implements the desired order and/or hierarchy of the one or more individual criteria C1, C2,....

In some embodiments, at least one predetermined criterion C1 comprises a safety criterion that the optical power of the second beam of light B2 propagating towards the eye E of the subject is lower than a prescribed safety limit. For example, this criterion may specifically consider a predefined power limit which the optical power of the beam of light that reaches the subject's eye must not exceed. This threshold may be set so that it corresponds to a specific power in terms of Watts or other applicable amplitude relevant for the operation configuration of the ophthalmic imaging instrument. For example, the prescribed safety limit may be set for a specific mode or imaging modality so that the optical power of the second beam of light B2 does not exceed 2 mW - at least within a safety margin - when reaching the eye E. This may specifically consider a modality in which a single light source 11 is contributing to the beam of light B2 imaging the eye.

Assuming the appropriate setting of the gain of the signal converter 15 and in an example of an analog signal routing implementation, the criterion may compare the amplitude of the output O to a voltage threshold. For a target optical power and a corresponding output voltage of 1 V, the safety limit may be set to 1.25 V which corresponds to a permissible power band of 25 % above the target power. If the output signal O is compared with the criterion C1 against a threshold voltage of 1.25 V and exceeds this, the protection circuit 16 can control the ophthalmic imaging instrument to power down the light source 11 as one exemplary form of preventing the delivery of the second beam of light B2 towards the eye of the subject. In this example, the condition that the output voltage O exceeds 1.25 V is equivalent to that the output O fails to satisfy the at least one predetermined criterion C1. Generally, this criterion C1 may be logically combined with one or more further criteria C2, C3....

In some embodiments, at least one predetermined criterion C2 comprises an image quality criterion that the optical power of the second beam of light B2 propagating towards the eye E of the subject is sufficient for acquiring an image of the eye having a signal-to-noise ratio above a prescribed level. In this context, the optical power may be considered from an image quality point of view and may specifically consider the fact that the incident optical power of a light beam determines the power of the reflected and/or refracted light which is detected as such as part of the very imaging. A lower incident optical power usually results in a lower power of any light coming from the object that is subject to imaging (e.g. a part of an eye, the retina, blood vessels, and the like). As this light is then detected and ultimately forms the base figure for compiling an image, the respective signal will be subject to noise and other disruptions as inherent to the involved hardware, detectors, optical systems and the like. Usually, the higher the power, the more advantageous the signal to noise ratio.

For example, this criterion may specifically consider a predefined power limit which the optical power of the beam of light that reaches the subject's eye should at least have. This threshold may be set so that it corresponds to a specific power in terms of Watts or other applicable amplitude relevant for the operation configuration of the ophthalmic imaging instrument. For example, the prescribed image quality limit may be set for a specific mode or imaging modality so that the optical power of the second beam of light B2 is at least reach 2 mW - at least with a tolerance margin - when reaching the eye E. This may specifically consider a modality in which a single light source 11 is contributing to the beam of light B2 imaging the eye.

Assuming the appropriate setting of the gain of the signal converter 15 and in an example of an analog signal routing implementation, the criterion may compare the amplitude of the output O to a voltage threshold. For a target optical power and a corresponding output voltage of 1 V, the image quality limit may be set to 0.75 V which corresponds to a permissible power band of 25 % below the target power. If the output signal O is compared with the criterion C2 against a threshold voltage of 0.75 V and is below this, the protection circuit 16 can control the ophthalmic imaging instrument to power down the light source 11 as one exemplary form of preventing the delivery of the second beam of light B2 towards the eye of the subject. In this example, the condition that the output voltage O is lower than 0.75 V is equivalent to that the output O fails to satisfy the at least one predetermined criterion C2. Generally, this criterion C2 may be logically combined with one or more further criteria C1, C3.... It is to be noted that any lower limit may be provided for avoiding the exposure of a subject's eye to radiation when this is not necessary or sensible, as even a light beam with an optical power below the image quality criterion is exposure and may impose stress onto the involved tissue.

Figure 3A shows a schematic view, including a circuit diagram, of a signal converter as part of an ophthalmic imaging instrument according to a more specific device embodiment of the present invention. Specifically, the signal converter 15' comprises an amplifier that is arranged to convert the input signal to an output voltage V using the gain G. For example, the input can be a current II that represents the signal which is output from the detector 14. In a detailed implementation example, the signal converter 15' may comprise an operational amplifier 151 in a current feedback amplifier (CFA) configuration which inputs the signal current at an input terminal 1511. The input current may be fed to an input and differential amplifier 1512 being coupled to a current/voltage converter 1513. An optional impedance converter 1514 may be provided for obtaining a low impedance at the output terminal 1515 outputting the output voltage VO. The configurations of operational amplifiers, instrumentation amplifiers, voltage buffers, current mirrors and the like are as such known.

However, in such general current/voltage converting implementations, the safety criterion can be set such that the output voltage V generated by the amplifier 151 is lower than a first threshold voltage V1 such that the optical power of the second beam B2 of light propagating towards the eye E of the subject is lower than the prescribed safety limit. Likewise, the image quality criterion can be set such that the output voltage V generated by the amplifier is higher than a second threshold voltage VT2, such that the optical power of the second beam of light propagating towards the eye of the subject is sufficient for acquiring the image of the eye having a signal-to-noise ratio above the prescribed level. The output voltage V of the amplifier 151 as the signal converted output O can then be directly compared to any one of the mentioned threshold voltages VT1 and VT2 by means of further amplifiers and/or logical gate circuits in order to ultimate effect the control for preventing any optical power to reach a subject's eye when the optical power of the beam of light propagating toward the eye does not comply with a given permissible power band. Thus, the provision of a signal conversion into a voltage allows for embodiments in which the protection circuit can be an electronic circuit that is arranged to shut down the light source if the output voltage is above the first threshold or below the second threshold. This configuration may provide for specific advantages in relation to relatively low circuit complexity and, with this, an associated relatively high operation reliability and/or relatively low manufacturing cost.

Generally and not only applicable to the above described current/voltage conversion, the detector 14 is arranged to receive the first beam of light B1 and the detector 14 can comprise a photodetector that is arranged to provide a signal current as the signal indicative of an optical power of the first beam of light. There may be provided an already described amplifier that converts the signal current into an output voltage or the signal current can be converted back to a voltage, e.g. by simple shunt, for subsequent analog to digital conversion (ADC). The handling of signals by means of currents may have the advantage that any parasitic resistances in the signal path do not alter the signal as the signal current is led through a well defined and closed path. Further, the current may be already generated as a base figure that directly - or at least to a relatively high degree - correlates with the optical power as the observable subject to measurement. For example, photodiodes may provide for a cost-efficient and reliable detection of optical power into a signal current. Yet further, the provision of the output signal in the form of a voltage allows the controller 12 to regulate the optical power of the beam of light B by minimizing an error signal which is indicative of a difference between the output voltage, generated e. g. by the amplifier 151, and a target voltage corresponding to the target optical power of the beam of light. The former target voltage would be for example 1 V in the context of the voltage examples given elsewhere in the present disclosure.

Figure 3B shows a schematic view, including a circuit diagram, of a signal converter as part of an ophthalmic imaging instrument according to a more specific device embodiment of the present invention. Specifically, the signal converter 15" comprises an amplifier configuration that is arranged to convert the input signal to an output voltage VO using the gain. In the present case, the detector comprises a photodiode 140 which is arranged to receive the beam of light B1, and which is fed from a supply and coupled via an input resistor 1521 to an input of an amplifier 1522. This input may be the negative input of amplifier 1522 in a transconductance amplifier configuration, in which the positive input may be coupled towards ground. The output of amplifier 1522 is coupled to the positive input of the instrumentation amplifier 1523, the output of which provides the basis for the output signal in the form of voltage VO. The negative input of instrumentation amplifier 1523 is coupled toward ground either directly or via a voltage divider or resistor depending on further implementation schemes. In this way, however, the amplifier 15" converts the current signal from the detector (comprising the photodiode 140) to the output voltage VO.

Figure 3B also shows possible implementations for a gain setting section. In such embodiments, the ophthalmic imaging instrument can generally comprise a gain setting section that is configured to vary the gain G and which can be controlled to change the gain G. Such embodiments can generally consider that the gain setting section may comprise a programmable voltage divider. More specifically and in a first alternative, there is considered the gain setting section 1530 in which the output of the instrumentation amplifier 1523 is fed back toward the negative input over a voltage divider against ground. Specifically, the voltage divider is formed by a first resistor 1531 toward ground at least one resistor 1532 which can be switched into the path between the amplifier's input and output via a switch 1533 (e.g. a transistor or an optic coupler). There may be provided a number of resistors and switches in parallel connection which is indicated by the dots "..." in Figure 3B.

The gain can this way be varied by means of opening and closing the switch(es) 1533,..., as the resistance and the voltage divider characteristics are changed accordingly. For example, switch 1533 can be closed while switch 1535 is open so as to form the voltage divider by resistors 1531 and 1532 which may set an effective gain of the amplifier 15" that corresponds to a setting in which a first power incident into the photodiode 140 yields some predefined value for the output voltage VO. In another case, for example for another imaging modality, switch 1533 can be open while switch 1535 is closed so as to form the voltage divider by resistors 1531 and 1534 which may set an effective gain of the amplifier 15" that corresponds to a setting in which a second power, which is different from the just mentioned first power, incident into the photodiode 140 yields the same predefined value for the output voltage VO. The values of the resistors 1531, 1532 and 1534 and the switching pattern applied to switches 1533 and 1535 determine the available gains in which the amplifier 15" can be set to operate.

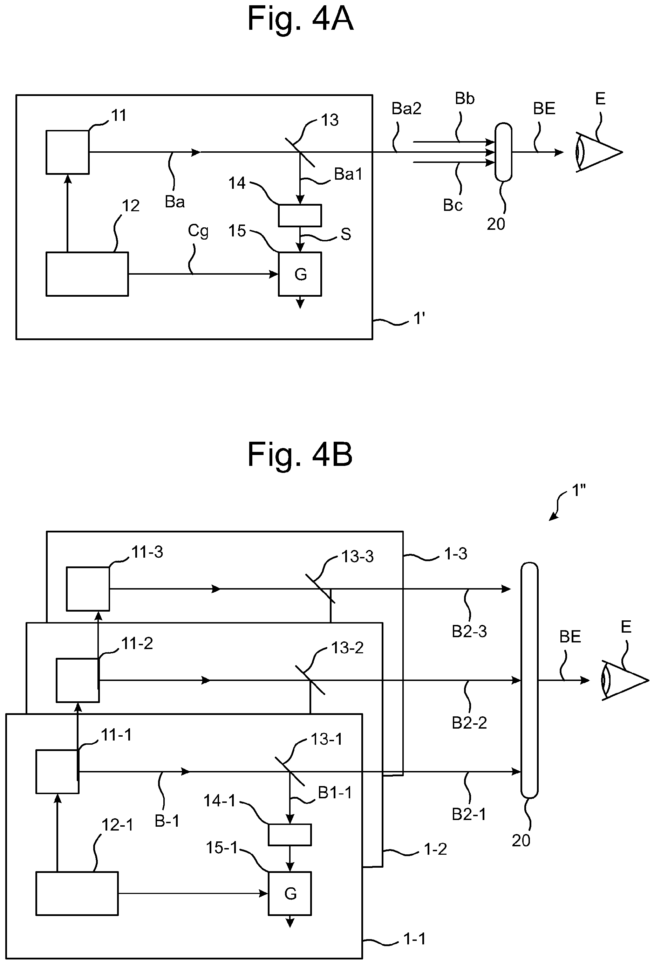

Figure 4A shows a schematic view of an ophthalmic imaging instrument for providing more than one imaging modality according to a more specific device embodiment of the present invention. Specifically, there is shown a multi-modal ophthalmic imaging instrument 1' which is operable in a plurality of imaging modalities. A contribution from the light source 11 to a power of light BE projected by the multi-modal ophthalmic imaging instrument 1' onto the eye E of the subject to image the eye varies among at least some of the imaging modalities and is set by the target optical power. In other words, the light source 11 provides only a part of light an optical power that propagates toward the eye E in the form of the second beam of light Ba2. It is noted that the disclosure in conjunction with Figure 1 may apply to the elements with same reference numerals, and that the beam of light Ba would correspond to the beam of light B in Figure 1, the beam of light Ba1 would correspond to the beam of light B1 in Figure 1, and that the beam of light Ba2 would correspond to the beam of light B2 in Figure 1. Further beams of light Bb, Bc, ... may also contribute to the power of light actually incident to the eye. ,.... The individual beams of light Ba2, Bb, Bc, ... can then be combined by a combing optics 20 so as to provide a focused single beam BE. This may in particular provide the possibility for medical and other assessments under different conditions and/or with different objectives.

For example, in a first imaging modality, the contribution of the light source 11 is relatively high, while other contributions are relatively low or even vanish. It is understood that the optical power budget permissible for a subject's eye is always the same - or at least similar-so that the power control and any protection mechanisms should consider the fact of the primary contribution by the light source 11. For example, the gain may be set such that a target output signal (voltage) is indicative of the target optical power which is exclusively provided by the light source 11 (e.g. the target voltage can be set to 1V with VT1 = 1.25V and VT2 = 0.75V). Then, in a second imaging modality, the contribution of the light source 11 is relatively low, while other contributions are also present. It is understood that the optical power budget permissible for a subject's eye is always the same - or at least similar - so that the power control and any protection mechanisms should consider the fact of the only partial contribution by the light source 11. For example, the gain may be set such that a target output signal (voltage) is indicative of the target optical power which is collectively provided by more than one light source (e.g. the target voltage is set again to 1 V but for target power which is only a third assuming that three light sources contribute to the beam of light BP to an equal degree).

In such embodiments, the controller 12 of the multi-modal ophthalmic imaging instrument 1' can be arranged to select the target optical power for the beam of light based on an imaging modality of the plurality of imaging modalities, in which imaging modality the ophthalmic imaging instrument is to operate. Following the above example, the target optical power can be set to 2 mW in the first example, when only one light source contributes, whereas the target optical power can be set to 0.66 mW in the second example, when three light sources contribute to the same degree. Generally, the plurality of imaging modalities may thus comprise at least a first imaging modality in which only one light source contributes to the light which propagates towards the eye of the subject, and a second imaging modality in which more than one light source contribute.

Figure 4B shows a schematic view of an ophthalmic imaging instrument for providing more than one imaging modality according to a more specific device embodiment of the present invention. Specifically, there is shown a multi-modal ophthalmic imaging instrument 1" which is operable in a plurality of imaging modalities and which comprises at least a plurality of light sources 11-1, 11-2, 11-3,..., each one being configured to contribute to a respective varying degree to light BP which propagates towards the eye E of the subject. In the shown a multi-modal ophthalmic imaging instrument 1" the respective degrees are dependent on one of the plurality of imaging modalities. It is noted that the multi-modal ophthalmic imaging instrument 1" may be configured by means of a plurality of ophthalmic imaging instruments 1 as described in conjunction with Figure 1. In such an implementation there also provided individual beam splitters 13-1, 13-2, 13-3, detectors 14-1, and the other corresponding components as described in greater detail in conjunction with Figure 1 and which are denoted by reference numerals ending with "-1", "-2" etc. The individual beams of light B2-1, B2-2, B2-3,... can then be combined by a combing optics 20 so as to provide a focused single beam BP. It is understood though that any other elements may again be provided separately or also in a more integrated manner. In case one or more of the light sources are provided in the form of laser light sources, the plurality of light sources can comprise a red laser, a green laser and a blue laser. In a first monochromatic or fluorescent imaging modality only one laser, e.g. the blue laser, operates, wherein in a second colour imaging modality all three lasers contribute in the sense of RGB imaging.

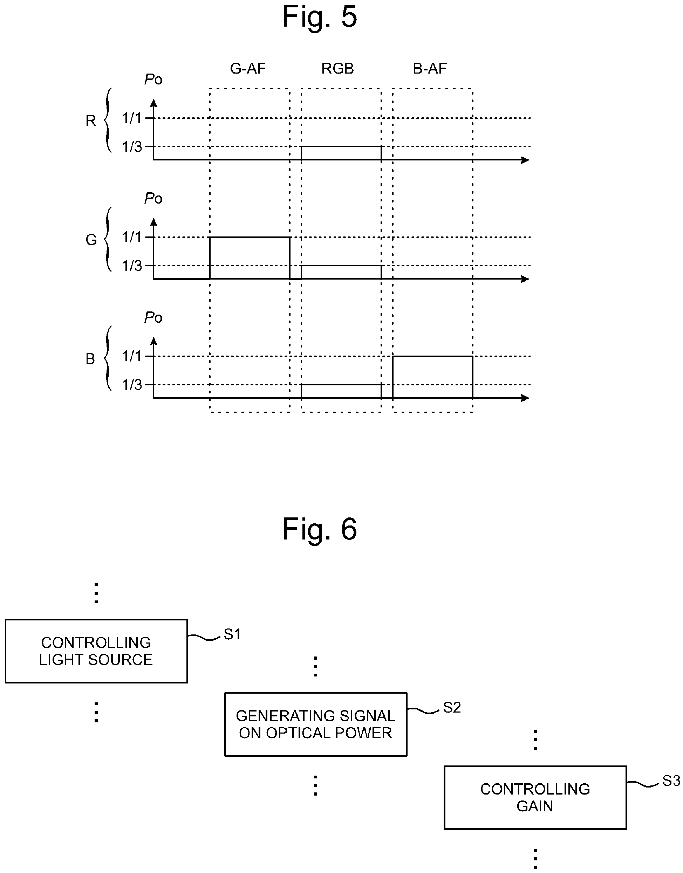

Figure 5 shows schematic views of optical powers for individual beams of light under different imaging modalities according to a more specific embodiment of the present invention. Specifically, there are shown respective target optical powers for three light sources R, G and B, wherein such light sources can comprise respective lasers that emit light in a red colour (R), a green colour (G) and in a blue colour (B). In a first imaging modality only one light source is active and is to use the full optical power budget by providing an optical power Po being denoted by 1/1. This imaging modality can be a green auto-fluorescence (G-AF) imaging modality, in which only green light is used. In such a modality, the gain can be set so that some desired output is produced, e.g. an output voltage of 1 V, when the optical power of the green light is at a given target power, e.g. 2 mW. In a second imaging modality all three light sources R, G and B are active and each light source can only use a fraction of the optical power budget. For example, each light source can be operated to provide only a third of the optical power 1/1, which is denoted by 1/3. This imaging modality can be a full colour or RGB imaging modality, in which white light is used. In such a modality, the gain can be set so that again the desired output is produced, e.g. an output voltage of 1 V, but when the optical power of each one of the contributing lights is at a respective fraction of the given target power, e.g. 0.66 mW. In a third imaging modality again only one light source is active and is to use the full optical power budget. This imaging modality can be a blue auto-fluorescence (B-AF) imaging modality, in which only blue light is used. In such a modality, the gain can be again set so that the same desired output is produced, e.g. an output voltage of 1 V, when the optical power of the blue light is at the (full) given target power of, for example, 2 mW.

Generally, the desired output can be different in different imaging modalities, although remaining in an output band which corresponds to the permissible power band of the light. Namely, for an assumed desired voltage of 1 V, the output band may be between 0.75 V and 1.25 V with a safety margin of 25% that governs the control of the ophthalmic imaging instrument to prevent delivery of the second beam of light towards the eye. Preferably, embodiments of the invention aim at normalizing the output to some same desired output for all imaging modalities, although the embodiments may certainly permit some tolerance in that the there is one desired output for one imaging modality and another desired output for another imaging modality, but all desired outputs are again withing a well-defined tolerance band such as the already mentioned output band. Specifically, the desired output voltages may be for example 1.0 V in a first imaging modality, but 1.1 V in a second imaging modality. However, the permissible output band may still be the same so that in both cases a variation beyond 1.25 V or below 0.75 V may lead to the control of preventing that light reaches the eye of the patient.

Figure 6 shows a flowchart of a general method embodiment of the present invention. Specifically, the method embodiment relates to operating an ophthalmic imaging instrument comprising a light source, a beam splitter arranged to split the beam of light into a first beam of light, and a second beam of light which propagates towards an eye of a subject, a detector arranged to receive the first beam of light and generate a signal indicative of an optical power of the first beam of light, and a signal converter having a variable gain for generating the output. Such an ophthalmic imaging instrument can be for example the ophthalmic imaging instrument 1 as described in conjunction with Figure 1, the ophthalmic imaging instrument 1' as described in conjunction with Figure 4A, the ophthalmic imaging instrument 1" and/or each constituent instrument 1-1, 1-2 or 1-3 as described in conjunction with Figure 4B. The method comprises a step S1 of controlling the light source to generate a beam of light of a target optical power; a step S2 of generating, using the signal converter, an output based on the signal indicative of the optical power; and a step S3 of controlling the gain of the signal converter using the target optical power such that the signal converter generates a predefined output when an optical power of the beam of light is the target optical power. The latter step S3 can be specifically performed when an imaging modality is changed for ophthalmic imaging instrument by setting the gain accordingly. For example, the gain may be set to a first gain in one or more imaging modalities of G-AF and B-AF as described in conjunction with Figure 5, and to a second gain in the imaging modality RGB as described in conjunction with Figure 5. As in such a case more light sources contribute in the latter RGB modality, the power budged needs to be shared by the light sources so that the output is amplified relatively stronger from the input by means of a relatively high gain, while in the former modalities, in which only a single light source my employ the full power budget, the output is amplified relatively weakly from the input by means of a relatively low gain. It is noted that these steps S1, S2, and S3 can be performed sequentially, repeatedly and/or concurrently depending on the actual implementation.

The forgoing has presented embodiments and details thereof as part of the present invention which can provide one or more advantages by improving power control mechanisms in ophthalmic imaging instruments that allow for the flexibility of providing a plurality of imaging modalities but maintain safety while not requiring an increase in system and/or implementation. While various embodiments of the present disclosure have been described above, it should be understood that they have been presented by way of example, not limitation. It will be apparent to persons skilled in the relevant art(s) that various changes in form and detail can be made therein. Thus, the above-described exemplary embodiments are not limiting.

## Claims

1. An ophthalmic imaging instrument (1, 1', 1") comprising:
a light source (11, 11-1,..);
a controller (12, 12-1,..) arranged to control the light source (11, 11-1,..) to generate a beam of light (B, Ba, B-1,..) of a target optical power;
a beam splitter (13, 13-1,..) arranged to split the beam of light (B, Ba, B-1,..) into a first beam of light (B1, Ba-1, B1-1,...), and a second beam of light (B2, Ba-2, B2-1,...) which propagates towards an eye (E) of a subject;
a detector (14, 14-1,..) arranged to receive the first beam of light (B1, Ba-1, B1-1,...) and
generate a signal (S) indicative of an optical power of the first beam of light (B1, Ba-1, B1-1,...); and
a signal converter (15, 15-1,...) arranged to generate an output based on the signal (S) indicative of the optical power, the signal converter (15, 15-1,...) having a variable gain (G) for generating the output,
wherein the controller (12, 12-1,..) is further arranged to control the gain (G) of the signal converter (15, 15-1,...) using the target optical power such that the signal converter (15, 15-1,...) generates a predefined output when an optical power of the beam of light (B, Ba, B-1,..) is the target optical power.

2. The ophthalmic imaging instrument (1, 1', 1") according to claim 1, wherein the predefined output is independent from the target optical power.

3. The ophthalmic imaging instrument (1, 1', 1") according to claim 1 or 2, further comprising a protection circuit (16) arranged to determine whether the output generated by the signal converter (15, 15-1,...) fails to satisfy at least one predetermined criterion, and to control the ophthalmic imaging instrument (1, 1', 1") to prevent delivery of the second beam of light (B2, Ba-2, B2-1,...) towards the eye (E) of the subject when the output fails to satisfy the at least one predetermined criterion.

4. The ophthalmic imaging instrument according to claim 3, wherein the at least one predetermined criterion comprises a safety criterion that the optical power of the second beam of light propagating towards the eye of the subject is lower than a prescribed safety limit.

5. The ophthalmic imaging instrument (1, 1', 1") according to claim 3 or 4, wherein the at least one predetermined criterion comprises an image quality criterion that the optical power of the second beam of light (B2, Ba-2, B2-1,...) propagating towards the eye (E) of the subject is sufficient for acquiring an image of the eye having a signal-to-noise ratio above a prescribed level.

6. The ophthalmic imaging instrument according to any one of claims 4 or 5, wherein the signal converter comprises an amplifier converting the signal to an output voltage using the gain.

7. The ophthalmic imaging instrument (1, 1', 1") according to claim 4 and 6, wherein the safety criterion is that the output voltage generated by the amplifier is lower than a first threshold such that the optical power of the second beam of light propagating towards the eye (E) of the subject is lower than the prescribed safety limit.

8. The ophthalmic imaging instrument (1, 1', 1") according to claim 5 and 6, wherein the image quality criterion is that the output voltage generated by the amplifier is higher than a second threshold, such that the optical power of the second beam of light (B2, Ba-2, B2-1,...) propagating towards the eye (E) of the subject is sufficient for acquiring the image of the eye having a signal-to-noise ratio above the prescribed level.

9. The ophthalmic imaging instrument (1, 1', 1") according to claims 7 and 8, wherein the protection circuit (16) is an electronic circuit arranged to shut down the light source (11, 11-1,...) if the output voltage is above the first threshold or below the second threshold.

10. The ophthalmic imaging instrument (1, 1', 1") according to any one of claims 6 to 9, wherein the detector (14, 14-1,...) arranged to receive the first beam of light (B1, Ba-1, B1-1,...) comprises a photodetector arranged to provide a signal current as the signal indicative of an optical power of the first beam of light (B1, Ba-1, B1-1,...), and wherein the amplifier converts the signal current into the output voltage.

11. The ophthalmic imaging instrument (1, 1', 1") according to any one of claims 6 to 10, wherein the controller (12, 12-1,..) is further arranged to regulate the optical power of the beam of light by minimizing an error signal which is indicative of a difference between the output voltage generated by the amplifier and a target voltage corresponding to the target optical power of the beam of light.

12. The ophthalmic imaging instrument (1, 1', 1") according to any one of claims 1 to 11, being a multi-modal ophthalmic imaging instrument operable in a plurality of imaging modalities, wherein a contribution from the light source (11, 11-1,...) to a power of light projected by the multi-modal ophthalmic imaging instrument onto the eye (E) of the subject to image the eye varies among at least some of the imaging modalities and is set by the target optical power, and the controller (12, 12-1,..) is further arranged to select the target optical power for the beam of light based on an imaging modality of the plurality of imaging modalities, in which imaging modality the ophthalmic imaging instrument (12, 12-1,..) is to operate.

13. The ophthalmic imaging instrument (1, 1', 1") according to claim 12, comprising a plurality of light sources (11-1, 11-2, 11-3,...) configured to contribute to a respective varying degree to light which propagates towards the eye (E) of the subject, the respective degrees being dependent on one of the plurality of imaging modalities.

14. The ophthalmic imaging instrument (1, 1', 1") according to claim 13, wherein the plurality of imaging modalities comprise at least a first imaging modality in which only one of said light sources (11-1, 11-2, 11-3,...) contribute to the light which propagates towards the eye of the subject, and a second imaging modality in which more than one of said light sources (11-1, 11-2, 11-3,...) contribute to the light.

15. A method of operating an ophthalmic imaging instrument comprising a light source, a beam splitter arranged to split the beam of light into a first beam of light, and a second beam of light which propagates towards an eye of a subject, a detector arranged to receive the first beam of light and generate a signal indicative of an optical power of the first beam of light, and a signal converter having a variable gain for generating the output, the method comprising the steps of
- controlling the light source to generate a beam of light of a target optical power;
- generating, using the signal converter, an output based on the signal indicative of the optical power; and
- controlling the gain of the signal converter using the target optical power such that the signal converter generates a predefined output when an optical power of the beam of light is the target optical power.
